# EUROPÄISCHE PATENTSCHRIFT

(11) **EP 3 499 511 B1**
(45) Veröffentlichungstag und Bekanntmachung des Hinweises auf die Patenterteilung: **15.05.2024**
(21) Anmeldenummer: 18210946.2
(22) Anmeldetag: 07.12.2018
(51) Int. Cl.: G16H 40/63, A61M 16/00, G16H 20/40, A61M 16/06, A61M 16/08

(54) **BEATMUNGSGERÄT MIT EINER VORRICHTUNG ZUR BERÜHRUNGSLOSEN ERFASSUNG VON BEDIENVORGÄNGEN EINES NUTZERS**
VENTILATOR WITH A DEVICE FOR THE TOUCHLESS DETECTION OF USER OPERATIONS
VENTILATEUR AVEC UN DISPOSITIF POUR LA DÉTECTION SANS CONTACT DES OPÉRATIONS DE L'UTILISATEUR

(30) Priorität: 18.12.2017 DE 102017011690
(43) Veröffentlichungstag der Anmeldung: 19.06.2019
(73) Patentinhaber: Löwenstein Medical Technology S.A., 2557 Luxembourg (LU)
(72) Erfinder: Schwaibold, Matthias, 76228 Karlsruhe (DE)
(74) Vertreter: Marx, Thomas

(56) Entgegenhaltungen:
- WO-A1-2017/089910
- WO-A2-2008/042219
- CN-U- 205 434 608
- US-A1- 2007 023 044
- US-A1- 2007 193 582

## Beschreibung

### Zusammenfassung der Erfindung

Die Erfindung ist in den beigefügten Ansprüchen definiert.

### Zusammenfassung der Offenbarung

Dokument US2007023044A1 offenbart ein Beatmungsgerät mit einer Projektionseinheit, welche ein Bild auf eine Oberfläche in der Nähe des Beatmungsgeräts projiziert. Der Nutzer kann mit dem projizierten Bild interagieren, um das Beatmungsgerät zu steuern.

Nachteil beim Stand der Technik ist, dass die gleichzeitig zu erfassende Informationsmenge nicht optimal für die menschliche Wahrnehmung aufbereitet werden kann. Außerdem kann bei einem Zugriff über mindestens eine Schnittstelle nur auf die Bedienelemente eines Computers oder ähnliches zurückgegriffen werden.

Die Interaktion des Nutzers mit den zuvor beschriebenen Anzeigeeinheiten wird immer komplexer, wodurch intelligente und/oder intuitive Bedienkonzepte erforderlich sind. Aufgabe der vorliegenden Erfindung ist es, eine gegenüber dem Stand der Technik verbesserte Vorrichtung und ein verbessertes Verfahren zur Erfassung von berührungslosen Bedienvorgängen eines Nutzers anzugeben. Die Aufgabe wird gelöst durch eine Vorrichtung zur Steuerung eines Beatmungsgerätes mittels einer Erfassungseinheit für Steuerbefehle, dadurch gekennzeichnet, dass die

Erfassungseinheit Steuerbefehle eines Nutzers in einem Erfassungsbereich der Erfassungseinheit erfasst, wobei die Erfassungseinheit zumindest einen Sensor für Steuerbefehle, einen Speicher, eine Vorrichtung zur Erzeugung digitaler Steuerbefehle und eine Schnittstelle zur Kopplung mit dem Beatmungsgerät und zur Versendung der Steuerbefehle an das Beatmungsgerät aufweist.

Die Vorrichtung ist auch dadurch gekennzeichnet, dass das Beatmungsgerät eine Schnittstelle zum Senden von Daten an die Erfassungseinheit aufweist.

Die Vorrichtung ist auch dadurch gekennzeichnet, dass die Erfassungseinheit als eine Kamera ausgebildet ist.

Die Vorrichtung ist auch dadurch gekennzeichnet, dass die Erfassungseinheit als eine 3D-Kamera oder TOF-Kamera mit einem Erfassungsbereich ausgebildet ist.

Die Vorrichtung ist auch dadurch gekennzeichnet, dass die Erfassungseinheit zur Verarbeitung und Auswertung im Erfassungsbereich erfasster Bilddaten oder Gesten ausgebildet ist.

Die Vorrichtung ist auch dadurch gekennzeichnet, dass die Erfassungseinheit momentane Bilddaten mit vorgegebenen Bilddaten zur Auswertung der Bewegung oder Geste eines Nutzers vergleicht, wobei die vorgegebenen Bilddaten in dem Speicher abgelegt sind.

Die Vorrichtung ist auch dadurch gekennzeichnet, dass anhand der im Erfassungsbereich erfassten Geste zumindest eine Anwendung, eine Interaktion und/oder eine Steuerung unterschiedlicher Funktionen des Beatmungsgerätes aktivierbar oder deaktivierbar sind bzw. ist.

Die Vorrichtung ist auch dadurch gekennzeichnet, dass die Erfassungseinheit mit einer Beleuchtungseinheit zur Beleuchtung des Erfassungsbereichs gekoppelt ist.

Die Vorrichtung ist auch dadurch gekennzeichnet, dass die Beleuchtungseinheit Licht im sichtbaren Bereich und/oder im Infrarotspektralbereich emittiert.

Die Vorrichtung ist auch dadurch gekennzeichnet, dass die Beleuchtungseinheit ein virtuelles Bedienelement in den Erfassungsbereich projiziert.

Die Vorrichtung ist auch dadurch gekennzeichnet, dass die Beleuchtungseinheit eine virtuelle Abbildung von Anzeige- und/oder Bedienelementen des Beatmungsgerätes in den Erfassungsbereich projiziert.

Die Vorrichtung ist auch dadurch gekennzeichnet, dass die Beleuchtungseinheit eine virtuelle Abbildung des Beatmungsgerätes in den Erfassungsbereich projiziert.

Die Vorrichtung ist auch dadurch gekennzeichnet, dass die Beleuchtungseinheit (32) Messwerte oder Auswertungen (36) des Beatmungsgerätes in den Erfassungsbereich (24) projiziert.

Vorrichtung (21) nach einem der vorhergehenden Ansprüche, dadurch gekennzeichnet, dass die Erfassungseinheit (22) in oder an dem Beatmungsgerät (20) angeordnet ist.

Die Vorrichtung ist auch dadurch gekennzeichnet, dass die Erfassungseinheit (22) als Fernbedienung ausgebildet ist.

Die Vorrichtung ist auch dadurch gekennzeichnet, dass die Beleuchtungseinheit (32) in oder an der die Erfassungseinheit (22) oder dem Beatmungsgerät (20) angeordnet ist.

Die Vorrichtung ist auch dadurch gekennzeichnet, dass die Erfassungseinheit einen Sprach-Sensor (25) umfasst und Sprachbefehle (23) erkennt und in digitale Steuerbefehle umwandelt und über die Schnittstelle (28) an das Beatmungsgerät (18, 20) sendet, wobei die Erfassungseinheit (22) ausgebildet ist Sprachbefehle (23) zu authentifizieren (30) und eine optische und/oder auditive und/oder grafische Rückantwort für den Nutzer generiert bevor der Steuerbefehl umgesetzt wird.

Die Aufgabe wird gelöst durch ein Verfahren zur Steuerung eines Beatmungsgerätes (20) mittels einer Erfassungseinheit (22) für Steuerbefehle, dadurch gekennzeichnet, dass die Erfassungseinheit (22) Steuerbefehle (23) eines Nutzers in einem Erfassungsbereich (24) der Erfassungseinheit (22) erfasst, wobei die Erfassungseinheit (22) zumindest einen Sensor (25) für Steuerbefehle (23), einen Speicher (29), eine Vorrichtung (27) zur Erzeugung digitaler Steuerbefehle und eine Schnittstelle (28) zur Kopplung mit dem Beatmungsgerät und zur Versendung der Steuerbefehle an das Beatmungsgerät (20) aufweist.

Die Aufgabe wird auch gelöst durch eine Vorrichtung zur Steuerung eines Beatmungsgerätes mittels einer Erfassungseinheit für Steuerbefehle, dadurch gekennzeichnet, dass die Erfassungseinheit analoge Steuerbefehle eines Nutzers in einem Erfassungsbereich der Erfassungseinheit erfasst, wobei die Erfassungseinheit zumindest einen Sensor für analoge Steuerbefehle, einen Speicher, einen Tiefpass und/oder einen Hochpassfilter, einen A/D-Wandler zur Erzeugung digitaler Steuerbefehle und eine Schnittstelle zur Kopplung mit dem Beatmungsgerät und zur Versendung der Steuerbefehle an das Beatmungsgerät aufweist.

Wenn die die Erfassungseinheit analoge Steuerbefehle eines Nutzers erfasst ist die Abtastfrequenz des A/D-Wandlers beispielsweise mindestens doppelt so groß wie die höchste vorkommende Frequenz im abgetasteten Signal des analogen Steuerbefehls.

Das erfindungsgemäße Beatmungsgerät und/oder Schlaftherapiegerät enthält eine virtuelle, räumlich (also dreidimensional) sichtbare Anzeigeeinheit und/oder Bedienelemente, die sich nicht direkt im Gerät befinden und damit auch aus der Ferne ablesbar und bedienbar sind.

Die Anzeigeeinheit und/oder Bedienelemente werden durch eine geeignete Bildgebungseinheit visualisiert, beispielsweise eine 3-D-Brille, halb-durchlässige 3-D-Brille, eine räumliche Bilder erzeugende Kontaktlinse, einen Monitor mit 3-D-Funktion (beispielsweise mit Shutter-Brille oder Polarisationsbrille) oder eine Projektionseinrichtung mit 3-D-Funktion. Die Bildgebungseinheit erzeugt eine räumliche Wahrnehmung von Information für mindestens einen Betrachter.

Die Bildgebungseinheit kann entweder direkt an das Beatmungsgerät angeschlossen werden, oder Bildgebungseinheit und Beatmungsgerät werden beide an eine Bilderzeugungseinheit angeschlossen, welche aus den Daten des Beatmungsgerät mit sehr geringer Zeitverzögerung die räumlichen Abbildungen erzeugt, oder die Bildgebungseinheit wird an die Bilderzeugungseinheit angeschlossen, welche räumliche Abbildungen aus zuvor empfangenen und gespeicherten Gerätedaten erzeugt. Bildgebungseinheit und Bilderzeugungseinheit können sich im selben Gehäuse befinden.

Die Kommunikation zwischen Beatmungsgerät und Bilderzeugungseinheit, sowie zwischen Bilderzeugungseinheit und Bildgebungseinheit erfolgt durch elektrische Signale oder elektromagnetische Wellen oder einen Lichtleiter. Möglich Kommunikationskanäle können sein: Internet, LAN, Mobilfunk, LPWA, USB, serielle Schnittstelle, VGA, HDMI, Displayport, Bluetooth etc.

Zusätzlich zur optischen Information wird bevorzugt auch eine akustische Information erzeugt, insbesondere Alarmtöne, menschliche Sprache, Sprache einer künstlichen Stimme, Musik oder akustisches Feedback bei Bedienen einer virtuellen Bedieneinheit, beispielsweise Rastgeräusche bei einem virtuellen Dreh-Druck-Knopf. Durch optische und akustische Ausgabe befindet sich der Nutzer in einer virtuellen Realität gemeinsam mit dem Beatmungsgerät.

Der Erfassungsbereich ist beispielsweise die Umgebung des Nutzers, also der Aktionsraum seiner Hände und Beine. Die Bildgebung muss jedoch nicht exakt in diesem Bereich erfolgen. Bei einer 3-D-Brille wird das Bild direkt vor den Augen erzeugt. Bei einer Projektion auf eine Leinwand ist der Erfassungsbereich Entfernt vom Aktionsraum des Nutzers. Erfindungsgemäß ist der Erfassungsbereich daher zumindest in einigen Fällen die Summe aus Bildgebungsbereich (Beispiel 3D-Brille oder Leinwand) und Aktionsraum.

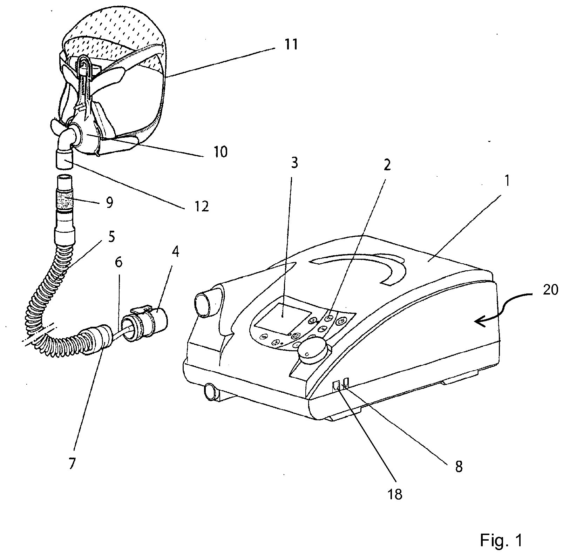

Fig. 1 zeigt den grundsätzlichen Aufbau einer Vorrichtung zur Beatmung. Im Bereich eines Gerätegehäuses (1) des Beatmungsgerätes (20), mit einer Atemgasquelle im Geräteinnenraum, sind ein Bedienelement (2) und ein Bedien-und Informationssystem (3) bestehend aus einer Anzeige (13), einer berührungsempfindlichen Eingabeeinheit (15) mit zumindest einem Bedienfeld (14) angeordnet. Über eine Kopplung (4) wird ein Verbindungsschlauch (5) angeschlossen. Entlang des Verbindungsschlauches (5) kann ein zusätzlicher Druckmessschlauch (6) verlaufen, der über einen Druckeingangsstutzen (7) mit dem Gerätegehäuse (1) verbindbar ist. Zur Ermöglichung einer Datenübertragung weist das Gerätegehäuse (1) zumindest eine Schnittstelle (8,18) auf. Ein Anfeuchter (30) kann zudem adaptiert werden.

Im Bereich einer dem Gerätegehäuse (1) abgewandten Ausdehnung des Verbindungsschlauches (5) ist ein Ausatmungselement (9) angeordnet. Ebenfalls kann ein Ausatemventil verwendet werden.

Das Beatmungsgerät (20) kann als Schlaftherapiegerät, als Anfeuchter oder Vernebler, als High-Flow-Gerät, als Anästhesiegerät, als klinisches- oder Heim- oder Notfall-Beatmungsgerät ausgebildet sein.

Fig. 1 zeigt darüber hinaus ein als Beatmungsmaske (10) ausgebildetes Patienten-Interface, das als Nasalmaske realisiert ist. Eine Fixierung im Bereich eines Kopfes eines Patienten kann über eine Kopfhaube (11) erfolgen. Im Bereich seiner dem Verbindungsschlauch (5) zugewandten Ausdehnung weist das Patienten-Interface (10) ein Kupplungselement (12) auf.

Über die Schnittstelle (8,18) kann die Eingabe und / oder Ausgabe von Daten, wie beispielsweise Totraumvolumen, erfolgen. Die Schnittstellen können kabelgebunden, als Infrarot-Schnittstelle, als Bluetooth-Schnittstelle oder USB realisiert sein. Bevorzugt ist auch ein Kartenschacht vorgesehen. Die Schnittstelle (8) kann auch als LAN-Schnittstelle oder als sonstige Schnittstelle zur Anbindung an das Internet ausgeführt sein. Im Bereich eines Gerätegehäuses kann ein Sauerstoffzuschaltventil an die Vorrichtung zur Beatmung adaptiert werden. Es ist denkbar, das Atemgas zusätzlich mit Sauerstoff anzureichern, um die Patientenversorgung zu verbessern.

Über die Schnittstelle (8) - beispielsweise als Kartenschacht oder USB ausgeführt - können auch therapiefremde Daten in das erfindungsgemäße Beatmungsgerät geladen werden beziehungsweise von diesem ausgeführt werden. Der Nutzer muss - werden vom Gerät externe Speichermedien erkannt - eine Abfrage im Bedienfeld bestätigen, woraufhin die Daten wahlweise im Bereich des Beatmungsgerätes gespeichert oder ausgeführt werden.

Das erfindungsgemäße Beatmungsgerät (20) ist so ausgelegt, dass es über einen Schlauch und ein Patienteninterface mit einem Patienten verbunden werden kann, um eine Beatmung bereitzustellen. Es umfasst eine Quelle für Atemgas, die beispielsweise als ein Elektromotor mit Gebläserad ausgebildet ist, und eine Einrichtung zur Ermittlung von Druck und/oder Fluss und/oder Volumen des Atemgases, sowie eine Steuereinheit (19), die so ausgelegt ist, dass sie für jeden Atemzyklus auf der Basis eines vorbestimmten Wertes für den Patienten und/oder auf Basis von Messsignalen für die Parameter Druck und/oder Flow und/oder Volumen einen Atemgasdruck bestimmt und die Quelle für Atemgas derart reguliert, dass der Atemgasdruck erzeugt wird.

Die Steuereinheit (19) ist ferner so ausgelegt, dass sie den aktuellen Druck und/oder Flow und/oder das Volumen von Atemgas bestimmt und den aktuellen Wert über das mit der Steuereinheit Bedien- und Informationssystem (3) darstellt. Die Steuereinheit (19) ist außerdem so ausgelegt, dass sie bezogen auf einen oder mehrere Parameter Trendveränderungen ihrer Berechnungen über der Zeit bestimmt, wobei die Trendveränderungen auf der Anzeige angezeigt werden können.

Weiterhin vergleicht die Steuereinheit (19) solche ParameterWerte, die von einem Benutzer vorgegeben wurden, beispielsweise obere und untere Druckgrenzen oder eine maximal tolerierbare Anzahl von Apnoen pro Zeiteinheit, oder eine maximal tolerierbare Leckage, mit den aktuellen Werten und generiert eine Benutzer-Information zu Abweichungen von der Vorgabe. Die Benutzerinformation wird bevorzugt über das Bedien- und Informationssystem (3) graphisch visualisiert.

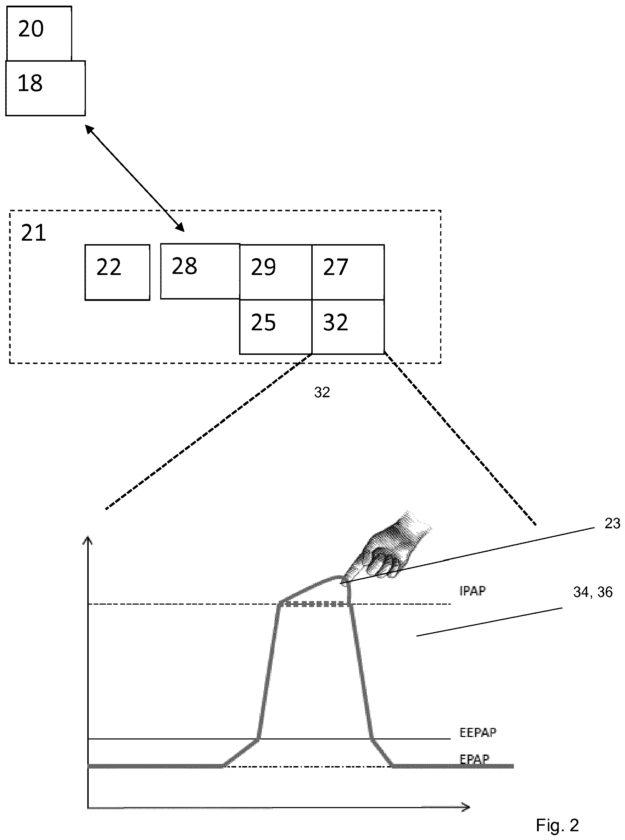

Figur 2 zeigt schematisch die Interaktion von Beatmungsgerät und der Vorrichtung zur Steuerung. Die Vorrichtung (21) zur Steuerung des Beatmungsgerätes (20) erfasst mittels der Erfassungseinheit (22) für Steuerbefehle, Steuerbefehle (23) eines Nutzers in einem Erfassungsbereich (24) der Erfassungseinheit (22). Die Erfassungseinheit (22) weist dazu zumindest einen Sensor (25) für Steuerbefehle (23), einen Speicher (29), eine Vorrichtung (27) zur Erzeugung digitaler Steuerbefehle und eine Schnittstelle (28) zur Kopplung mit dem Beatmungsgerät und zur Versendung der Steuerbefehle an das Beatmungsgerät (20) aufweist.

Das Beatmungsgerät weist eine korrespondierende Schnittstelle (18) zu der Erfassungseinheit (22) auf. Beispielsweise versenden die Schnittstellen wechselseitig Daten. Beispielsweise verwenden die Schnittstellen Bluetooth oder andere Funkübertragungs-Methoden.

Die Erfassungseinheit (22) ist mit einer Beleuchtungseinheit (32) zur Beleuchtung des Erfassungsbereichs (24) gekoppelt oder weist diese auf. Die Beleuchtungseinheit (32) emittiert Licht im sichtbaren Bereich und/oder im Infrarotspektralbereich. Die Beleuchtungseinheit (32) projiziert ein virtuelles Bedienelement (33) und/oder eine virtuelle Abbildung von Anzeige- und/oder Bedienelementen (34) in den Erfassungsbereich (24).

Die Erfassungseinheit (22) zur Verarbeitung und Auswertung im Erfassungsbereich (24) erfasster Bilddaten oder Gesten (23) ausgebildet ist. Die Erfassungseinheit (22) vergleicht für die Erkennung der Gesten beispielsweise momentane Bilddaten (30) mit vorgegebenen Bilddaten (31) zur Auswertung der Bewegung oder Geste (23) eines Nutzers, wobei die vorgegebenen Bilddaten (30) in dem Speicher (29) abgelegt sind.

Anhand der im Erfassungsbereich (24) erfassten Geste (23) wird zumindest eine Anwendung, eine Interaktion und/oder eine Steuerung unterschiedlicher Funktionen des Beatmungsgerätes (20) aktiviert oder deaktiviert. Vorliegend stellt der Nutzer durch eine Wischgeste den IPAP-Druck ein.

Vorteilhafterweise sind mittels der Erfassungseinheit ein Gegenstand und/oder ein Nutzer und/oder von diesem ausgeführte Gesten und/oder Bedienvorgänge dreidimensional erfassbar. Beispielsweise wird somit eine Bewegung einer Hand oder eines Fingers dreidimensional erfasst, welche beispielweise einer virtuellen Betätigung einer Funktion des Beatmungsgerätes entspricht. Dabei kann es sich um die Erfassung eines Bedienvorgangs mit einer Geste, wie beispielsweise ein Hin- und Herbewegen eines Fingers oder eine Wischbewegung oder Öffnen der Hand als Zoombewegung, handeln.

Die Erfassungseinheit wandelt die erfasste Geste oder Bewegung in ein entsprechendes elektrisches Signal um und übermittelt dieses an das Beatmungsgerät, welches die, entsprechend der in dem elektrischen Signal enthaltene, Information dem gewünschten Bedienvorgang zuordnet.

In einer Ausführungsform wird das Beatmungs- oder Schlaftherapiegerät von der Bildgebungseinheit in einer Zwillingsabbildung in seiner Form und mit seinen Anzeige- und Bedienelementen genau wie das echte Beatmungsgerät dargestellt, um dem Nutzer dieselben Informationen und/oder Handlungsmöglichkeiten zu bieten, als würde er sich in Sichtweite und/oder Reichweite des Gerätes befinden.

In einer bevorzugten Ausführungsform werden die Messwerte, die sonstigen Informationen aus dem Gerät und / oder über den Patienten sowie die Bedienelemente zusätzlich oder statt der originalgetreuen Abbildung erweitert dargestellt, um dem Nutzer die Wahrnehmung der Information und die Handlungsmöglichkeiten dreidimensional in einer optimierten Weise darzustellen, wie es am Gerät mit seinem zweidimensionalen Display nicht möglich wäre.

Die Anzeigeelemente zeichnen sich dadurch aus, dass bestimmte besonders relevante Informationen nicht nur vergrößert, sondern auch räumlich vor oder über weniger relevanten Informationen dargestellt werden. Dabei wird durch eine Anzeigelogik entschieden, welche Anzeigeelemente im jeweils augenblicklichen Kontext wie relevant sind.

Dabei wird bei mindestens einem vom Beatmungsgerät oder der Bilderzeugungseinheit erkannten besonderen und/oder kritischen Muster im Atemfluss des Patienten ein Atemflusssignal räumlich vor mindestens einem anderen Signal dargestellt, beispielsweise dem Drucksignal oder Leckagesignal. Das Atemflusssignal selbst kann dabei entweder zweidimensional (beispielsweise flache Kurve) oder dreidimensional (beispielsweise Kurve mit einer Dicke, Breite und Tiefe) dargestellt werden.

Bevorzugt wird bei mindestens einem vom Beatmungsgerät oder der Bilderzeugungseinheit erkannten besonderen und/oder kritischen Muster im Leckageverlauf der Maske oder des Patienten ein Leckagesignal räumlich vor mindestens einem anderen Signal dargestellt, beispielsweise dem Drucksignal oder Volumensignal. Das Leckagesignal selbst kann dabei entweder zweidimensional (beispielsweise flache Kurve) oder dreidimensional (beispielsweise Kurve mit einer Dicke, Breite und Tiefe) dargestellt werden.

Bevorzugt wird zu mindestens einem dargestellten Signal ein Zeitverlauf von mehr als einer Minute derart dargestellt, dass sich der neueste Zeitabschnitt vor den älteren Zeitabschnitten des Signals befindet. Ist die Darstellung des vordersten Zeitabschnittes vollständig gefüllt, so wird dieser mindestens um ein wahrnehmbares Stück nach hinten versetzt, und räumlich davor werden die aktuellen Werte des Signals ein einem neuen Zeitabschnitt dargestellt.

Bevorzugte Signale, die räumlich zueinander angeordnet dargestellt werden, sind: Atemfluss des Patienten, Leckage, Therapiedruck, Atemvolumen, Atemfrequenz, Zeitdauer der Inspiration, Zeitdauer der Exspiration, Verhältnis der Zeitdauern zueinander, Sauerstoffsättigung im Blut, CO2-Konzentration im Blut, CO2-Konzentration im Atemfluss, O2-Konzentration im Atemfluss, Pulsrate, Pulssignal, Pulswellensignal, Triggersignal, Art des Atemzuges, erwartetes Atemvolumen, Atemkontur, Widerstand der Atemwege, Atemanstrengung, Atemexkursion, Atem-/Schnarchgeräusche, Atemereignisse (beispielsweise Apnoen), Temperatur, Luftfeuchte, Benutzungszeiten und -dauern des Beatmungsgerätes, Einstellungen des Beatmungsgerätes oder von Zubehörkomponenten. Sie Signale werden als Kurvenverläufe, als aktueller Zahlenwert, als räumliche Markierung, als Zahlenwertverlauf, als Balkendiagramm, als Säulendiagramm, als Kreisdiagramm oder Netzdiagramm dargestellt. Dabei können entweder aktuelle Daten dargestellt werden, oder Daten aus der Vergangenheit, die beispielsweise im Beatmungsgerät oder in der Bilderzeugungseinheit oder einem Datenträger oder einem Server gespeichert sind.

Zahlenwerte können dreidimensional in einer räumlichen Tabelle dargestellt werden, bei der die Größe des Wertes oder seine Position (vorne / hinten, oben / unten) oder seine Farbe eine Interpretation beinhalten. BEISPIELSWEISE können alle Werte, die sich oberhalb des Normalbereiches befinden, räumlich gesehen oberhalb einer Normalposition dargestellt werden. Werte, die sich unterhalb des Normalbereiches befinden, können räumlich gesehen unterhalb einer Normalposition dargestellt werden. Bevorzugt wird in relevanten Situationen, in denen eine Handlung des Nutzers möglich und/oder vorteilhaft ist, eine zusätzliche erklärende Information räumlich dargestellt, Diese zusätzliche erklärende Information besteht typischerweise aus Text oder Ziffern oder Symbolen. Besonders bevorzugt wird eine Verknüpfung der zusätzlichen Information mit den Signalen hergestellt, in denen die relevanten Situationen erkannt wurden.

Bei einer erkannten erhöhten Leckage wird in einer Ausführungsform der Text "Hohe Leckage" in räumlichem Bezug zum Leckagesignal dargestellt und der Text wird mit dem Signal über einen Pfeil verknüpft.

Bei einer erkannten niedrigen Sauerstoffsättigung wird in einer Ausführungsform der Text "Niedrige Sättigung" in räumlichem Bezug zum SpO2-Signal dargestellt und der Text wird mit dem Signal über einen Pfeil verknüpft.

Bei einer erkannten niedrigen Atemvolumen oder Apnoe wird in einer Ausführungsform der Text "Niedriges Volumen" in räumlichem Bezug zum Volumen- oder Atemfluss-Signal dargestellt und der Text wird mit dem Signal über einen Pfeil verknüpft.

Weitere relevante Situationen können sein: Diskonnektion, zu hoher Druck, zu niedriger Druck, zu großes Volumen, zu hohe Temperatur, zu niedrige Temperatur, ungünstige Zusammensetzung des Atemgases (CO2, 02, Feuchte etc.), spezielle Aktion, beispielsweise Betätigen eines Bedienelementes) durch den Patienten oder durch mindestens einen Nutzer, Hypopnoen, Schnarchen, Veränderung der Blutgase, Veränderung der Pulsfrequenz, Veränderung des Herzschlagvolumens, Veränderung des Atemvolumens, Veränderung des Atemmusters, Veränderung der Atemfrequenz, Veränderungen beim Zubehör, beispielsweise dem Befeuchter.

Bei besonders kritischen Situationen wird die dargestellte erklärende Information zusätzlich in einer anderen Farbe dargestellt als bei weniger kritischen Situationen, beispielsweise in Orange oder Rot. Zusätzlich besteht die Möglichkeit, die erklärende Information in kritischen Situationen optisch zu verändern, beispielsweise weiter vorn darzustellen, zu vergrößern, blinken zu lassen, pulsieren zu lassen, sich zyklisch hin- und her-bewegen zu lassen.

Die erklärende Information kann dabei auch aus zusätzlichen Datenquellen stammen, die nicht im Beatmungsgerät verfügbar sind und nicht notwendigerweise mit einer optischen Verknüpfung zu den Signalen aus dem Gerät dargestellt werden. Dies können beispielsweise der Name mindestens einer Person, beispielsweise des Patienten, ein Gewicht, eine Körpergröße, ein Datum, eine Uhrzeit, Informationen aus der Krankengeschichte, Informationen aus der Gerätegeschichte oder Nachrichten von anderen Personen als dem aktuellen Nutzer sein.

Die erklärende Information kann auch aus wertenden Symbolen bestehen, beispielsweise Emoticons oder Haken oder +/- Symbolen oder Ausrufezeichen, die dem Nutzer eine schnelle Bewertung der dargestellten Information ermöglichen oder einer detaillierteren Informationsebene vorgeschaltet sind. Zur Bewertung des Information kann auch eine Einfärbung des Hintergrundes oder die Darstellung eines Hintergrundbildes in einer virtuellen Umgebung dienen, beispielsweise eine positiv oder weniger positiv besetzte Szene.

Erklärende Informationen können auch Handlungsempfehlungen sein. BEISPIELSWEISE bestimmte Änderungen am Beatmungsgerät oder seinen Einstellungen oder seinem Zubehör oder die Kontaktierung einer betreuenden Person oder Serviceperson bzw. -organisation. Bevorzugt werden Kontaktdaten dieser Personen oder Organisationen im Rahmen der erklärenden Information angezeigt. In einer alternativen Ausführungsform ist die Bildgebungseinheit mindestens teilweise transparent, so dass beispielsweise der Arzt oder Patientenbetreuer den Patienten und das Beatmungsgerät real sehen kann und zusätzliche erklärende Information für ihn eingeblendet wird.

Zusätzlich gibt es in einer Ausführungsform die Möglichkeit, die erklärende Situation durch einen Schalter vermehrt zuzuschalten, um beispielsweise auf besonders dafür präparierten Daten einen Lernmodus oder Tutorialmodus zu durchlaufen. Im Lernmodus wird dem Nutzer beispielsweise erklärt, wie bestimmte Informationen dargestellt werden und interpretiert werden können. Außerdem werden ihm seine Handlungsmöglichkeiten erklärt, wie er mit der dargestellten Information und/oder mit dem virtuellen und echten Beatmungsgerät interagieren kann. In einem Tutorialmodus wird zusätzlich eine Lernkontrolle durchgeführt, in der der Nutzer Aufgaben beantworten und/oder lösen muss, bei denen keine unmittelbare Interaktion mit der Beatmung eines echten Patienten stattfindet.

Bevorzugt kann dabei die Bilderzeugungseinheit unterschiedliche Informationsmengen und -darstellungsformen anbieten je nach Nutzer oder Nutzertyp. Besonders bevorzugt werden für einen ärztlichen Nutzer oder einen Patientenbetreuer mehr Informationen angeboten als für einen Patienten. Auch der Lern- und Tutorialmodus ist auf die jeweils für den Nutzer verfügbare Informationsmenge zugeschnitten.

Zusätzlich bietet die Bildgebungseinheit die Möglichkeit, menschliche Personen in räumlicher Darstellung einzublenden. Dies geschieht bevorzugt gleichzeitig mit der Darstellung von Signalen oder anderen Informationen aus dem Beatmungsgerät. Bevorzugt werden ein medizinischer Experte und ein Patient wechselseitig eingeblendet, so dass sie sich über die aktuelle Therapiesituation in einer Art und Weise unterhalten können, als ob sie sich in Sichtweite zueinander befinden würden. Zusätzlich kann eine künstliche menschliche Person eingeblendet werden, die dem Nutzer erklärende Informationen zeigt oder zu ihm spricht. Diese virtuelle Person übernimmt beispielsweise eine Trainerfunktion, eine Assistenzfunktion oder eine Warngeber-Funktion. Bevorzugt erklärt die virtuelle Person dem Nutzer die Interpretation der dargestellten Information und/oder seine Handlungsmöglichkeiten, um die Informationsdarstellung oder die Einstellungen des realen Beatmungsgerätes in seiner virtuellen Umgebung zu verändern.

Insbesondere ist vorgesehen, dass die virtuelle Person dem Nutzer, bevorzugt dem Patienten, bestimmte Abläufe am Gerät, am Befeuchter oder an der Atemmaske in der virtuellen Realität erklärt. Besonders bevorzugt die Ankopplung, Abkopplung, Befüllung, Entleerung und Reinigung des Befeuchters und das korrekte Aufsetzen und Verstellen der Maske. Dabei besitzt die Bilderzeugungseinheit eine Information über den verwendeten Gerätetyp, Befeuchtertyp und/oder Maskentyp, damit die virtuelle Trainingsperson dieselben Komponenten verwendet wie der Patient. Erfindungsgemäß ist es vorgesehen, dass der Nutzer, beispielsweise Arzt oder Patient, die Informationsdarstellung während der Benutzung der Bildgebungseinheit verändern kann. BEISPIELSWEISE kann er bestimmte Informationen einblenden und ausblenden, vergrößern oder verkleinern, verschieben oder zwischen Darstellungsformen umschalten, beispielsweise räumliche Kurvendarstellung und räumliche Tabelle. Außerdem kann er bestimmte Hilfs- oder Erklärfunktionen ein- und ausschalten, beispielsweise das Einblenden von Texten oder einer virtuellen Person. Des Weiteren kann er Zusatzinformationen, wie beispielsweise Daten aus der Krankengeschichte, Namen, Alter, Größe oder eine wechselseitige Abbildung von Patient und Betreuer (virtuelle Visite) aktivieren und deaktivieren oder in ihrer Darstellungsform und -größe verändern. Dies gilt auch für akustische Informationen und Sprachausgaben, die beispielsweise ein- und ausgeschaltet oder in ihrer Lautstärke verändert oder in ihrer Art / Sprache verändert werden können. Außerdem ist vorgesehen, dass mindestens eine zusätzliche reale Person durch eine Aktion des Nutzers kontaktiert und bevorzugt mit in die virtuelle Realität eingebunden werden, beispielsweise Patientenbetreuer, Patient, Angehörige, Serviceperson, Arzt, Krankenschwester, Physiotherapeut, Techniker etc. Dazu wird zu dieser Person eine Daten- und/oder Sprachverbindung aufgebaut und sie bekommt eine zusätzliche räumliche und akustische oder konventionell zweidimensionale Darstellung der aktuell für sie relevanten oder verfügbaren Informationen und Bedienmöglichkeiten angeboten.

Die Änderung der Informationsdarstellung erfolgt typischerweise durch mindestens eine Handbewegung des Nutzers, bevorzugt in Verbindung mit einer Kopfbewegung des Nutzers. Dabei wird mindestens ein virtuelles Bedienelement vom Nutzer angeschaut und bedient. Alternativ kann auch ein reales Bedienelement betätigt werden, beispielsweise eine Maus, Tastatur, Joystick, Schieberegler, Drehregler oder ein Touchscreen, welches an die Bilderzeugungseinheit angeschlossen ist oder mit ihre Daten austauschen kann. Ein mögliches Bedienelement ist auch ein Datenhandschuh, der die Hand- und Fingerbewegungen des Nutzers erkennt und bevorzugt durch Aktoren ein haptisches Feedback geben kann, beispielsweise das Gefühl des Betätigens eines Schiebereglers oder Drehreglers. Alternativ kann die Änderung der Informationsdarstellung auch über ein Sprachkommando erfolgen.

Erfindungsgemäß ist es vorgesehen, dass der Nutzer, beispielsweise Arzt oder Patient, die Einstellungen oder den Zustand des Beatmungsgerätes während der Benutzung der Bildgebungseinheit verändern kann. Insbesondere ist vorgesehen, dass die Beatmungstherapie gestartet oder gestoppt werden kann, dass die Leistung des Befeuchters verändert werden kann und mindestens ein Therapiedruck oder eine Therapiefrequenz oder eine Komfortfunktion beeinflusst werden kann. Außerdem ist vorgesehen, dass mindestens eine Triggerfunktion oder Druckänderungsgeschwindigkeit oder der Zielwert für mindestens eine Beatmungsfunktion, beispielsweise ein Zielvolumen, beeinflusst werden kann. Außerdem ist vorgesehen, dass mindestens eine Meldung oder ein Alarm am Gerät quittiert oder beendet werden kann. Außerdem ist vorgesehen, dass mindestens ein Selbsttest ausgelöst oder eine Datenübertragung gestartet werden kann, entweder vom Gerät zu einer Gegenstelle (beispielsweise Auslesen von Therapie- oder Servicedaten) oder von einer Gegenstelle zum Gerät (neue Konfiguration, Update mindestens einer auf dem Gerät ausgeführten Software). Außerdem ist vorgesehen, dass ein Test zur Vermessung des Beatmungssystems (Widerstand, Compliance und/oder Leckage) ausgelöst werden kann, beispielsweise durch Anfahren bestimmter Testdrücke in vordefinierten Zuständen des Zubehörs (beispielsweise getrennt vom Patient oder vollständig mit dem Patienten verbunden).

Bevorzugt kann dabei die Bilderzeugungseinheit unterschiedliche Möglichkeiten zur Änderung der Einstellungen oder des Zustandes des Beatmungsgerätes anbieten je nach Nutzer oder Nutzertyp. Besonders bevorzugt werden für einen ärztlichen Nutzer oder einen Patientenbetreuer mehr Änderungsmöglichkeiten angeboten als für einen Patienten.

Die Änderung der Einstellungen oder des Zustandes des Beatmungsgerätes erfolgt typischerweise durch mindestens eine Handbewegung des Nutzers, bevorzugt in Verbindung mit einer Kopfbewegung des Nutzers. Dabei wird mindestens ein virtuelles Bedienelement vom Nutzer angeschaut und bedient. Alternativ kann auch ein reales Bedienelement betätigt werden, beispielsweise eine Maus, Tastatur, Joystick, Schieberegler, Drehregler oder ein Touchscreen, welches an die Bilderzeugungseinheit angeschlossen ist oder mit ihre Daten austauschen kann. Ein mögliches Bedienelement ist auch ein Datenhandschuh, der die Hand- und Fingerbewegungen des Nutzers erkennt und bevorzugt durch Aktoren ein haptisches Feedback geben kann, beispielsweise das Gefühl des Betätigens eines Schiebereglers oder Drehreglers. Alternativ kann die Änderung der Informationsdarstellung auch über ein Sprachkommando erfolgen.

Das Smartphone wird per Kabel oder Bluetooth oder mit dem Gerät verbunden, der Arzt in der Klinik hat eine "Weinmann-Ärzte-App", und kann dann bei der Visite am Patientenbett einfach anordnen "IPAP auf 20", während er Blick und mindestens eine Hand beim Patient behalten kann. Die App würde dann Sprachbefehle in herkömmliche Fernsteuer-Kommandos umwandeln und ans Gerät senden.

Erfindungsgemäß kann eine Sprach- und/oder Gestenerkennung in der Cloud (im Internet) erfolgen. Die Daten einer Sprach- und/oder Gestenerkennung werden vor Ort erfasst und zur Auswertung in die Cloud gesendet. Dort steht mehr Rechenleistung zur Verfügung, und die Erkennungsalgorithmen können laufend optimiert werden - besser als auf lokalen Endgeräten. Die Erfassungseinheit (22) kann also aus mehreren Komponenten bestehen, die sich nicht alle vor Ort, sondern teilweise auch entfernt auf einem Server befinden können.

Die Erfassungseinheit (22) und/oder die Vorrichtung (21) zur Steuerung eines Beatmungsgerätes weist dazu zumindest eine Schnittstelle zur Übertragung der Daten einer Sprach- und/oder Gestenerkennung zu einem Computersystem oder Server auf. Über diese Schnittstelle werden die ausgewerteten Daten beispielsweise auch wieder empfangen.

Erfindungsgemäß sind mittels der Sprach- und/oder Gestenerkennung, in der dargestellten Weise, auch eine Überprüfung und Quittierung von Alarmen möglich. Erfindungsgemäß sind mittels der Sprach- und/oder Gestenerkennung, auch Serviceprozesse, beispielsweise das Aufspielen von Firmware, Versendung von Daten, Auslösen bestimmter Selbsttests möglich.

Für ein Steuergerät, welches mit einem Beatmungsgerät verbunden wird, und in der Lage ist, Sprachbefehle in Steuerbefehle umzuwandeln. Mit Sprach-Sensor, Speicher-Einheit, Verarbeitungs-Einheit, Schnittstelle zum Gerät, .... Die Verarbeitungseinheit enthält entsprechende Sicherheits- und Plausibilitäts-Logiken. Mit Authentifizierungslogik, Rückantwort grafisch / per Sprache an den Bediener.

Beim Verfahren zur berührungslosen Erfassung von Nutzern und von diesen ausgeführten Gesten und/oder Bedienvorgängen werden erfindungsgemäß mittels einer optischen Erfassungseinheit der Nutzer und von diesen ausgeführten Gesten und/oder Bedienvorgänge dreidimensional erfasst.

## Patentansprüche

1. Vorrichtung (21) zur Steuerung eines Beatmungsgerätes (20) mittels einer Erfassungseinheit (22) für Steuerbefehle, wobei die Erfassungseinheit (22) Steuerbefehle (23) eines Nutzers in einem Erfassungsbereich (24) der Erfassungseinheit (22) erfasst, wobei die Erfassungseinheit (22) zumindest einen Sensor (25) für Steuerbefehle (23), einen Speicher (29), eine Vorrichtung (27) zur Erzeugung digitaler Steuerbefehle und eine Schnittstelle (28) zur Kopplung mit dem Beatmungsgerät und zur Versendung der Steuerbefehle an das Beatmungsgerät (20) aufweist, wobei die Erfassungseinheit (22) mit einer Beleuchtungseinheit (32) zur Beleuchtung des Erfassungsbereichs (24) gekoppelt ist und die Beleuchtungseinheit (32) Licht im sichtbaren Bereich und/oder im Infrarotspektralbereich emittiert, wobei das Beatmungsgerät zumindest eine Anzeige (13) umfasst, **dadurch gekennzeichnet, dass** die Vorrichtung zumindest eine Bildgebungseinheit umfasst, wobei die Bildgebungseinheit dazu eingerichtet ist, Messwerte, sonstige Informationen und/oder Bedienelemente zusätzlich erweitert und dreidimensional darzustellen und wobei besonders relevante Informationen räumlich und vergrößert dargestellt werden und durch eine Anzeigelogik entschieden wird, welche Informationen im jeweiligen Augenblick wie relevant sind.

2. Vorrichtung (21) nach Anspruch 1, **dadurch gekennzeichnet, dass** das Beatmungsgerät eine Schnittstelle (18) zu der Erfassungseinheit (22) aufweist.

3. Vorrichtung (21) nach einem der vorhergehenden Ansprüche, **dadurch gekennzeichnet, dass** die Erfassungseinheit (22) als eine Kamera ausgebildet ist und zur Verarbeitung und Auswertung im Erfassungsbereich (24) erfasster Bilddaten oder Gesten (23) ausgebildet ist.

4. Vorrichtung (21) nach einem der vorhergehenden Ansprüche, **dadurch gekennzeichnet, dass** die Erfassungseinheit (2) als eine 3D-Kamera oder TOF-Kamera mit einem Erfassungsbereich (24) ausgebildet ist.

5. Vorrichtung (21) nach einem der vorhergehenden Ansprüche, **dadurch gekennzeichnet, dass** die Erfassungseinheit (22) momentane Bilddaten (30) mit vorgegebenen Bilddaten (31) zur Auswertung der Bewegung oder Geste (23) eines Nutzers vergleicht, wobei die vorgegebenen Bilddaten (30) in dem Speicher (29) abgelegt sind.

6. Vorrichtung (21) nach einem der vorhergehenden Ansprüche, **dadurch gekennzeichnet, dass** anhand der im Erfassungsbereich (24) erfassten Geste (23) zumindest eine Anwendung, eine Interaktion und/oder eine Steuerung unterschiedlicher Funktionen des Beatmungsgerätes (20) aktivierbar oder deaktivierbar sind bzw. ist.

7. Vorrichtung (21) nach einem der vorhergehenden Ansprüche, **dadurch gekennzeichnet, dass** die Beleuchtungseinheit (32) ein virtuelles Bedienelement (33) in den Erfassungsbereich (24) projiziert.

8. Vorrichtung (21) nach einem der vorhergehenden Ansprüche, **dadurch gekennzeichnet, dass** die Beleuchtungseinheit (32) eine virtuelle Abbildung von Anzeige- und/oder Bedienelementen (34) des Beatmungsgerätes und eine virtuelle Abbildung des Beatmungsgerätes (35) in den Erfassungsbereich (24) projiziert.

9. Vorrichtung (21) nach einem der vorhergehenden Ansprüche, **dadurch gekennzeichnet, dass** die Beleuchtungseinheit (32) Messwerte oder Auswertungen (36) des Beatmungsgerätes in den Erfassungsbereich (24) projiziert.

10. Vorrichtung (21) nach einem der vorhergehenden Ansprüche, **dadurch gekennzeichnet, dass** die Erfassungseinheit (22) in oder an dem Beatmungsgerät (20) angeordnet ist.

11. Vorrichtung (21) nach einem der vorhergehenden Ansprüche, **dadurch gekennzeichnet, dass** die Erfassungseinheit (22) als Fernbedienung ausgebildet ist.

12. Vorrichtung (21) nach einem der vorhergehenden Ansprüche, **dadurch gekennzeichnet, dass** die Beleuchtungseinheit (32) in oder an der die Erfassungseinheit (22) oder dem Beatmungsgerät (20) angeordnet ist.

13. Vorrichtung (21) nach einem der vorhergehenden Ansprüche, **dadurch gekennzeichnet, dass** die Erfassungseinheit einen Sprach-Sensor (25) umfasst und Sprachbefehle (23) erkennt und in digitale Steuerbefehle umwandelt und über die Schnittstelle (28) an das Beatmungsgerät (18, 20) sendet, wobei die Erfassungseinheit (22) ausgebildet ist Sprachbefehle (23) zu authentifizieren (30) und eine optische und/oder auditive und/oder grafische Rückantwort für den Nutzer generiert bevor der Steuerbefehl umgesetzt wird.

## Claims

1. A device (21) for controlling a ventilator (20) by means of a detection unit (22) for control commands, wherein the detection unit (22) detects control commands (23) of a user in a detection region (24) of the detection unit (22), wherein the detection unit (22) comprises at least one sensor (25) for control commands (23), a memory (29), a device (27) for generating digital control commands, and an interface (28) for coupling to the ventilator and for sending the control commands to the ventilator (20), wherein the detection unit (22) is coupled to a lighting unit (32) for lighting the detection region (24) and the lighting unit (32) emits light in the visible range and/or in the infrared spectral range, wherein the ventilator comprises at least one display (13), **characterized in that** the device comprises at least one imaging unit, wherein the imaging unit is configured to additionally represent measured values, other information, and/or control elements in an expanded and three-dimensional manner and wherein particularly relevant information is represented spatially and enlarged and a display logic decides which information is relevant at the respective moment.

2. The device (21) according to claim 1, **characterized in that** the ventilator comprises an interface (18) to the detection unit (22).

3. The device (21) according to one of the preceding claims, **characterized in that** the detection unit (22) is designed as a camera and is designed to process and evaluate image data or gestures (23) detected in the detection region (24).

4. The device (21) according to one of the preceding claims, **characterized in that** the detection unit (2) is designed as a 3D camera or TOF camera with a detection region (24).

5. The device (21) according to one of the preceding claims, **characterized in that** the detection unit (22) compares current image data (30) with predefined image data (31) in order to evaluate the movement or gestures (23) of a user, wherein the predefined image data (30) are stored in the memory (29).

6. The device (21) according to one of the preceding claims, **characterized in that** at least one application, an interaction, and/or a control of different functions of the ventilator (20) can be activated or deactivated based on the gesture (23) detected in the detection region (24).

7. The device (21) according to one of the preceding claims, **characterized in that** the lighting unit (32) projects a virtual control element (33) into the detection region (24).

8. The device (21) according to one of the preceding claims, **characterized in that** the lighting unit (32) projects a virtual depiction of display and/or control elements (34) of the ventilator and a virtual depiction of the ventilator (35) into the detection region (24).

9. The device (21) according to one of the preceding claims, **characterized in that** the lighting unit (32) projects measured values or evaluations (36) of the ventilator into the detection region (24).

10. The device (21) according to one of the preceding claims, **characterized in that** the detection unit (22) is arranged in or on the ventilator (20).

11. The device (21) according to one of the preceding claims, **characterized in that** the detection unit (22) is designed as a remote control.

12. The device (21) according to one of the preceding claims, **characterized in that** the lighting unit (32) is arranged in or on the detection unit (22) or ventilator (20).

13. The device (21) according to one of the preceding claims, **characterized in that** the detection unit comprises a voice sensor (25) and recognizes voice commands (23) and converts same into digital control commands and sends same via the interface (28) to the ventilator (18, 20), wherein the detection unit (22) is designed to authenticate (30) voice commands (23) and generates a visual and/or auditory and/or graphic response for the user before the control command is implemented.

## Revendications

1. Dispositif (21) destiné à commander un ventilateur (20) au moyen d'une unité de détection (22) pour des ordres de commande, dans lequel l'unité de détection (22) détecte des ordres de commande (23) d'un utilisateur dans une zone de détection (24) de l'unité de détection (22), dans lequel l'unité de détection (22) présente au moins un capteur (25) pour des ordres de commande (23), une mémoire (29), un dispositif (27) destiné à générer des ordres de commande numériques et une interface (28) destinée à être couplée au ventilateur et à envoyer des ordres de commande au ventilateur (20), dans lequel l'unité de détection (22) est couplée à une unité d'éclairage (32) destinée à éclairer la zone de détection (24) et l'unité d'éclairage (32) émet de la lumière dans la plage visible et/ou dans la plage spectrale infrarouge, dans lequel le ventilateur comporte au moins un affichage (13), **caractérisé en ce que** le dispositif comporte au moins une unité d'imagerie, dans lequel l'unité d'imagerie est configurée pour agrandir et représenter en trois dimensions des valeurs de mesure, d'autres informations et/ou des éléments de contrôle et dans lequel des informations particulièrement pertinentes sont représentées spatialement et grossies et une logique d'affichage permet de déterminer à chaque instant le degré de pertinence des différentes informations.

2. Dispositif (21) selon la revendication 1, **caractérisé en ce que** le ventilateur présente une interface (18) avec l'unité de détection (22).

3. Dispositif (21) selon l'une des revendications précédentes, **caractérisé en ce que** l'unité de détection (22) est conçue comme une caméra et conçue pour le traitement et l'évaluation de données d'image ou de gestes (23) détectés dans la zone de détection (24).

4. Dispositif (21) selon l'une des revendications précédentes, **caractérisé en ce que** l'unité de détection (2) est conçue comme une caméra 3D ou comme une caméra TOF avec une zone de détection (24).

5. Dispositif (21) selon l'une des revendications précédentes, **caractérisé en ce que** l'unité de détection (22) compare des données d'image actuelles (30) avec des données d'image prédéfinies (31) pour l'évaluation du mouvement ou du geste (23) d'un utilisateur, dans lequel les données d'image prédéfinies (30) sont enregistrées dans la mémoire (29).

6. Dispositif (21) selon l'une des revendications précédentes, **caractérisé en ce que** les gestes (23) détectés dans la zone de détection (24) permettent d'activer ou de désactiver au moins une application, une interaction et/ou une commande de différentes fonctions du ventilateur (20).

7. Dispositif (21) selon l'une des revendications précédentes, **caractérisé en ce que** l'unité d'éclairage (32) projette un élément de contrôle virtuel (33) dans la zone de détection (24).

8. Dispositif (21) selon l'une des revendications précédentes, **caractérisé en ce que** l'unité d'éclairage (32) projette une image virtuelle d'éléments d'affichage et/ou de contrôle (34) du ventilateur et une image virtuelle du ventilateur (35) dans la zone de détection (24).

9. Dispositif (21) selon l'une des revendications précédentes, **caractérisé en ce que** l'unité d'éclairage (32) projette des valeurs de mesure ou des évaluations (36) du ventilateur dans la zone de détection (24).

10. Dispositif (21) selon l'une des revendications précédentes, **caractérisé en ce que** l'unité de détection (22) est disposée dans ou sur le ventilateur (20).

11. Dispositif (21) selon l'une des revendications précédentes, **caractérisé en ce que** l'unité de détection (22) est conçue comme une télécommande.

12. Dispositif (21) selon l'une des revendications précédentes, **caractérisé en ce que** l'unité d'éclairage (32) est disposée dans ou sur l'unité de détection (22) ou le ventilateur (20).

13. Dispositif (21) selon l'une des revendications précédentes, **caractérisé en ce que** l'unité de détection comporte un capteur vocal (25) et reconnaît des ordres vocaux (23) et transforme ceux-ci en ordres de commande numériques et envoie ceux-ci au ventilateur (18, 20) par le biais de l'interface (28), dans lequel l'unité de détection (22) est conçue pour authentifier (30) des ordres vocaux (23) et génère une réponse optique et/ou auditive et/ou graphique pour l'utilisateur avant la transformation de l'ordre de commande.
